# EUROPÄISCHE PATENTSCHRIFT

(11) **EP 1 100 539 B1**
(45) Veröffentlichungstag und Bekanntmachung des Hinweises auf die Patenterteilung: **20.11.2002**
(21) Anmeldenummer: 99952335.0
(22) Anmeldetag: 06.08.1999
(51) Int. Cl.: A61K 45/06, A61K 31/60, A61K 31/37, A61K 31/365, A61K 31/35, A61K 31/19, A61K 31/195, A61P 9/10

(54) **WIRKSTOFFKOMBINATION INSBESONDERE ZUR PROPHYLAXE UND THERAPIE VON ISCHÄMISCHEN ORGANSCHÄDEN UND REPERFUSIONSSYNDROMEN**
COMBINATION OF ACTIVE SUBSTANCES, ESPECIALLY FOR THE PROPHYLAXIS AND THERAPY OF ISCHAEMIC ORGANIC LESIONS AND REPERFUSION SYNDROMES
COMBINAISON DE PRINCIPES ACTIFS, EN PARTICULIER POUR LA PROPHYLAXIE ET LA THERAPIE DE LESIONS ORGANIQUES ISCHEMIQUES ET DES SYNDROMES DE REPERFUSION

(30) Priorität: 06.08.1998 DE 19835674; 25.09.1998 DE 19844116
(43) Veröffentlichungstag der Anmeldung: 23.05.2001
(73) Patentinhaber: Vascular Biotech GmbH, 80331 München (DE)
(72) Erfinder: NEES, Stephan, D-81375 München (DE); JUCHEM, Gerd, D-81379 München (DE)
(74) Vertreter: Grund, Martin, Dr.
(86) Internationale Anmeldenummer: DE9902478
(87) Internationale Veröffentlichungsnummer: WO00007578

(56) Entgegenhaltungen:
- WO-A-96/35453

## Beschreibung

Die Erfindung betrifft eine Wirkstoffkombination (verabreichbar beispielsweise als Infusions- bzw. Injektionslösung, als Tablette, Inhalat oder in beliebiger anderer Darreichungsform) als Pharmazeutikum insbesondere zur Prophylaxe und Therapie von während partieller oder globaler Ischämie entstehenden und nach Reperfusion sich manifestierenden Organschäden und anderen Syndromen.

Eine in allen Bereichen eines Organs physiologisch regulierte Durchblutung ist die wichtigste Voraussetzung für die Lebensfähigkeit der betreffenden Gewebezellen: Sauerstoff und Nährsubstrate werden antransportiert und sichern die Deckung des ständig vorhandenen Energiebedarfs - Kohlendioxid, Metabolite und Wärme, die den energieliefernden und strukturerhaltenden Stoffwechsel der Zellen zu hemmen drohen, werden abtransportiert.

Vor diesem Hintergrund ist verständlich, daß Durchblutungsstörungen aller Art aufgrund des sich rasch einstellenden Energiedefizits die Funktionsfähigkeit der Organe bis hin zum Absterben bedrohen. Manche Organe, wie z. B. Skelettmuskeln, können selbst globale Ischämien bei 37°C mehrere Stunden, andere, wie z. B. das Gehirn, nur wenige Minuten überleben. Eine herausragende Rolle nimmt das ebenfalls wenig ischämietolerante Herz ein, da von dessen Leistungsfähigkeit (und damit von der ausreichenden Durchblutung der Koronararterien) die Blutversorgung aller anderen Körperorgane abhängt. Die entsprechende Grundlagenforschung konzentrierte sich aus diesem Grund besonders auf das Herz.

Kommt die Organdurchblutung nach einer Phase der Ischämie wieder in Gang, dann besteht paradoxerweise gerade im Augenblick der Redurchblutung und kurz danach ein erhebliches Gefährdungspotential für die Gewebestrukturen. Dieses den Pathologen schon lange bekannte Phänomen wird als "Reperfusionssyndrom" bezeichnet (Lit. 1). Schon in diesem Begriff kommt zum Ausdruck, daß unter Umständen sehr viele verschiedene pathogene Einflüsse ursächlich verantwortlich gemacht werden können. Im Brennpunkt heutiger Forschung zur Entstehung primärer myokardialer Reperfusionsschäden stehen die verschiedenen Gewebe des Koronarsystems, und an erster Stelle das vaskuläre Endothelgewebe als Auskleidung der Blutgefäße und als eigentlicher Blutbehälter des Körpers. Dieses bisher im Hinblick auf seine Funktionalität in den verschiedenen Blutgefäßarten noch wenig differenziert charakterisierte Gewebe erscheint besonders durch verschiedene Oxidantien aus aktivierten Leukozyten, Makrophagen und / oder Histiozyten stark gefährdet. Entzündungsmediatoren aus derartigen aktivierten Blut- und / oder Gewebezellen sollen außerdem die Permeabilität des Endothels erhöhen. Dies könnte zur Entstehung von ausgedehnten interstitiellen Ödemen führen, die wiederum die Mikrozirkulation bedrohen würden.

Heute vor allem am Herzen angewendete therapeutische Maßnahmen gegen das Energiedefizit betreffen die Ausschaltung elektrischer und mechanischer Arbeitsleistungen des Organs sowie eine globale Abkühlung. Erstere wird durch kontinuierliche oder diskontinuierliche Anwendung diverser hyperkaliämischer ("kardioplegischer") kristalloider oder bluthaltiger Perfusionslösungen erreicht, letztere, indem man diese meist auf 4°C vorkühlt und das perfundierte Organ damit auf eine Temperatur unter 17°C (4-17°C) einstellt (Lit. 2). Beide Maßnahmen laufen auf eine Reduktion des Energiestoffwechsels hinaus. Spezielle Zusätze wie Adenosin, Pyruvat und Ribose (siehe EP-B-0 108 820) oder Glucose und Insulin (Lit. 3) zum hyperkaliämischen Perfusionsmedium sollen die Verfügbarkeit myokardialer ATP-Reserven verbessern. Eine hohe Pufferkapazität, vorzugsweise mit Hilfe von Histidin, soll die ischämisch bedingte Ansäuerung des Myokards einschränken. Praktiziert wurden auch Maßnahmen zur Anhebung der myokardialen Glykogenkonzentration bzw. Purinverbindungen wie z. B Adenosin 12 h vor Beginn einer kardioplegisch geführten Herzoperation. Stark proklamiert wird neuerdings von einigen Kliniken auch die Anwendung der besonders teuren Blutkardioplegie (Lit. 4), also die Perfusion intraoperativ stillzustellender Organe mit hyperkaliämisch gemachtem Blut aus dem übrigen Patientenkreislauf. Diese neue Technik wurde nicht zuletzt vor allem auch zur Verhinderung von Reperfusionssehäden eingesetzt. Dabei steht vor Augen, daß Blut viele antioxidativ wirksame Verbindungen enthält, sich durch eine hohe Pufferkapazität auszeichnet, eine hohe Sauerstoffkapazität aufweist, und nicht zuletzt aufgrund des durch die Plasmaproteine ausgeübten onkotischen Drucks einen hohen, der Bildung myokardialer Ödeme entgegenstehenden osmotischen Druck entfaltet. Notwendigerweise muß das Blut im peripheren Kreislauf aber ständig über Membranen oxygeniert werden, eine Methode, die schon für sich zu einer Aktivierung von Leukozyten und Thrombozyten führen kann, die dann bei einer späteren Reperfusion gefährlich werden können. Inwieweit solche Maßnahmen auch allein schon zur Verstopfung von Mikrogefäßen und damit bei Reperfusion z. B. der koronaren Mikrozirkulation auch der koronaren Kapillaren führen ("capillary plugging"), ist noch wenig erforscht. Die Anwesenheit eventuell vorstimulierter Leukozyten und Thrombozyten im Koronarsystem erscheint im Lichte der oben beschriebenen Erkenntnisse über die endothelial bedingte Pathogenese von Reperfusionsschäden jedenfalls sehr bedenklich. Diese Interpretation befindet sich in Übereinstimmung mit Literaturbefunden, nach denen Maßnahmen zur spezifischen Elimination von Leukozyten aus Vollblut die beobachtbaren Reperfusionsschäden erheblich verringert haben (Lit. 5). Aus praktischen Gründen kann dieser Aufwand im klinischen Routinebetrieb jedoch nicht durchgeführt werden. Ganz ähnliche Beobachtungen und Limitationen gelten auch im Hinblick auf die Blockierung endothelialer oder leukozytärer Adhäsionsmoleküle mit spezifischen Antikörpern. Diskutiert wird außerdem Allopurinol als Kardioprotektivum, das als Inhibitor der ischämisch aus Xanthindehydrogenase gebildeten Xanthinoxidase die im Augenblick der Reperfusion über dieses Enzym laufende Superoxidradikalproduktion verhindern soll. Allerdings wird durch diesen Inhibitor auch die endogen ablaufende Harnsäureproduktion stark vermindert, was im Hinblick auf die antioxidativen Eigenschaften dieses Purinkörpers bedenklich ist (Allopurinol selbst hat keine antioxidative Eigenwirkung). Zu nennen sind schließlich auch noch verschiedene, durchweg aber nur auf eine bessere Kontrolle konservativer Therapieansätze hinauslaufende Prinzipien der Abwicklung des Reperfusionsverfahrens selbst, die erstmalig von Buckberg (Lit. 6) formuliert wurden.

WO-A-96/35453 beschreibt die Behandlung von atherosklerotischen vaskulären Krankheiten mit einer Kombination von einem Endothelin Antagonist und/oder einem Hemmstoff der Endothelin Synthase mit Steroidhormonen und/oder mit einem NO-Donor und/oder NO-Substrat und optional mit einem Hemmstoff der Cyclooxygenose.

Überblickt man alle klinisch verfügbaren Maßnahmen gegen ischämisch bedingte Organschäden, so läßt sich festhalten, daß Hypothermie und hyperkaliämische Perfusionstechnik durch Senkung des Energiebedarfs betroffener Organe zwar große Fortschritte auf operativem, vor allem kardiochirurgischem Sektor ermöglicht haben, die damit exzielbaren Überlebenszeiten von 2-6 h aber immer noch zu niedrig liegen, um viele operative Probleme voll in den Griff zu bekommen. Gegen die in der Praxis sehr häufig auftretenden, strukturellen und fünktionellen Reperfusionsschäden gab es bis heute überhaupt noch keine kausale Therapiemöglichkeit.

Der Erfindung liegt zunächst die Aufgabe zugrunde, diese bisher noch unbekannten pathogenetischen Mechanismen zumindest soweit aufzuklären, daß die Auswahl bzw. Bereitstellung spezifisch und umfassend wirkender Pharmakakombinationen zur Prophylaxe und Therapie struktureller und funktioneller Reperfusionsschäden in zuvor ischämischen Organgeweben ermöglicht wird.

Dieser initialen Zielsetzung folgend gelang es uns erstmalig, Endothelzellen aus den kleinsten Venen (= venoläre Endothelzellen) des Koronarsystems von Tier und Mensch zu isolieren und ihrer funktionellen Spezialisierung nach zu charakterisieren. Die im folgenden geschilderten Versuchsgruppen 1-3 geben auch in hierfür speziell entwickelte methodische Ansätze Einblick. Weiterhin gelang es, Spezies-autologe Blutzellarten (Lymphozyten, Monozyten, Granulozyten und Thrombozyten) in besonders reinen Fraktionen zu gewinnen und für die Versuche einzusetzen.

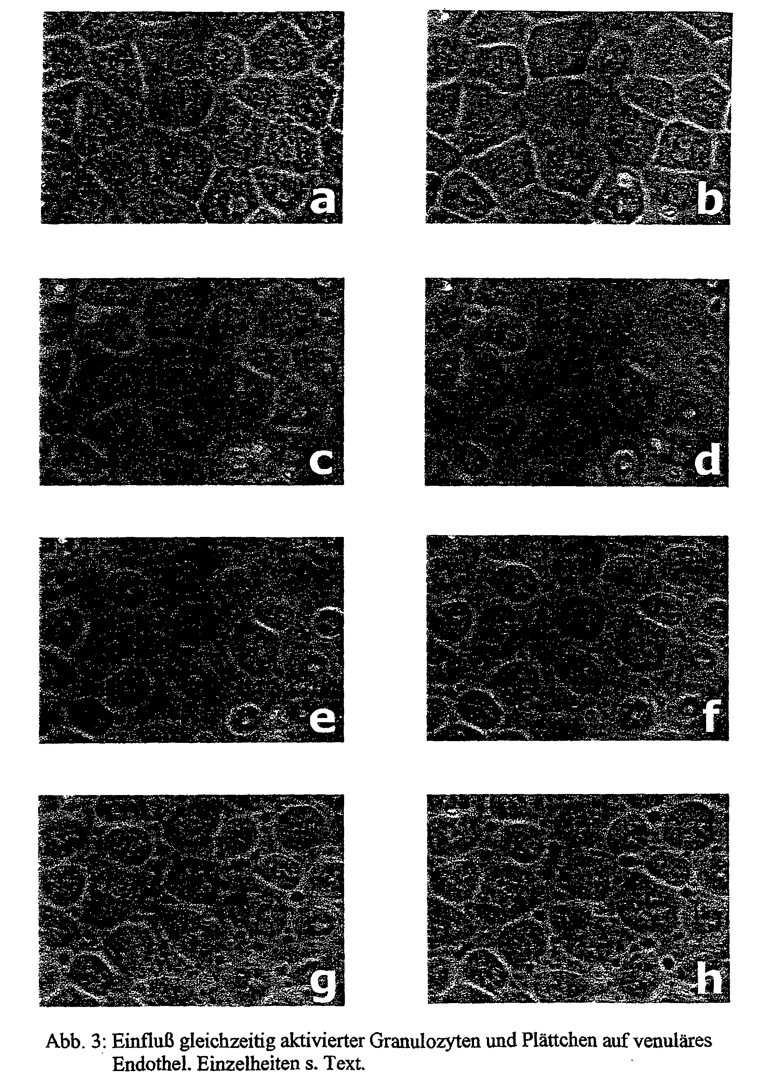

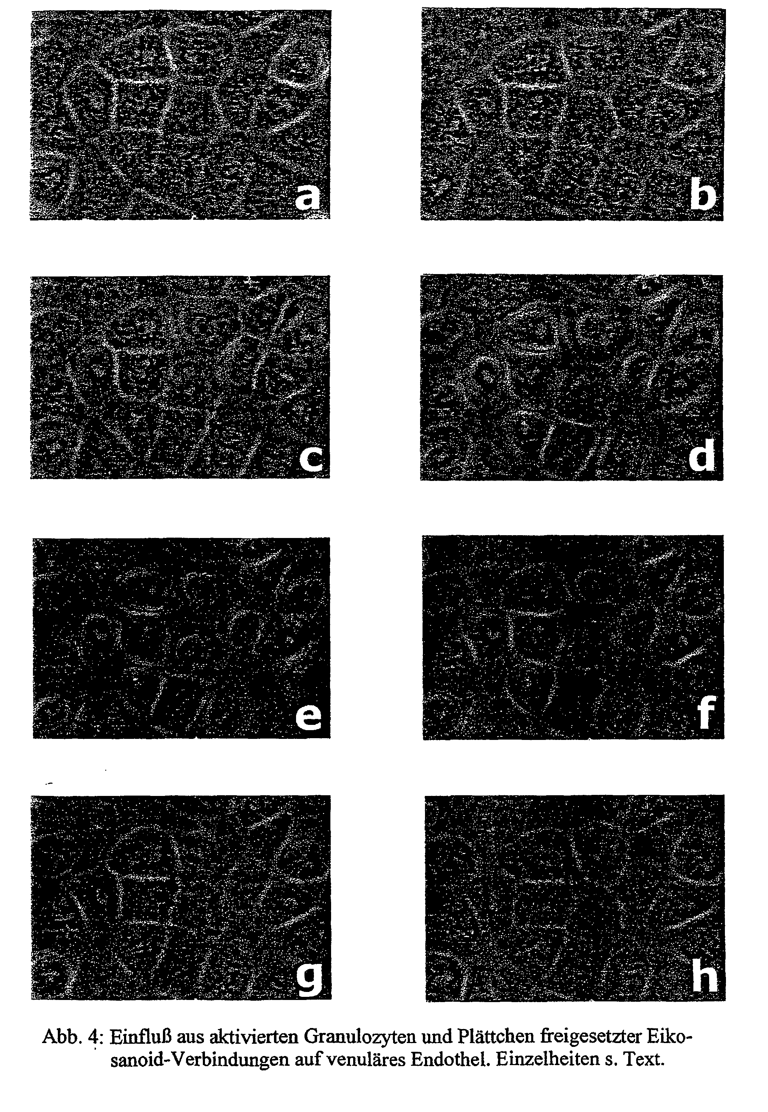

Aufgrund systematischer Studien (vgl. Versuchsgruppen 1-3) wurde entdeckt, daß Endothelzellen venolären Ursprungs über eine überraschende Kontraktilität verfügen. Endothelzellen aus anderen Blutgefäßtypen (Kapillaren, Arteriolen, große Blutgefäße) besitzen diese Kontraktilität nicht. Zeitraffervideomikroskopische Serienbilder (Abb. 3 und 4) zeigen besonders direkt, daß der jeweilige Kontraktionszustand der venolären Endothelschicht entscheidend ist für die Dichtigkeit der Venolen. Pathogenetisch von höchstem Interesse ist nun, daß gleichzeitig aktivierte Leukozyten und Thrombozyten in metabolischer Interaktion Arachidonsäuremetabolite freisetzen können, die schon in geringster Konzentration eine rasche Kontraktion venolärer Endothelzellen bewirken und so in vivo interstitielle Ödeme induzieren. Einen wichtigen Aspekt dieser metabolischen Interaktion bildet die Freisetzung von Vorläufersubstanzen aus aktivierten Thrombozyten, die in Anwesenheit von Leukozyten zu endothel-konstriktiv wirksamen Verbindungen weiterverstoffwechselt werden.

Der Erfindung liegt weiterhin die Aufgabe zugrunde, eine Wirkstoffkombination (verabreichbar beispielsweise als Infusions- bzw. Injektionslösung, als Tablette, Inhalat oder in beliebiger anderer Darreichungsform) zur Prophylaxe und Therapie von ischämischen Organschäden und Reperfusionssyndromen bereitzustellen, die damit auch zur Prophylaxe und Therapie von Mikrozirkulationsstörungen aller Art (z. B. im Rahmen von arteriosklerotischen Prozessen, Thrombosen, Bindegewebserkrankungen wie Parodontose, Verbrennungen und Vaskulitiden aller Art), allen Formen des Kreislaufschocks, der Eklampsie und zur Unterstützung einer therapeutischen Immunsuppression geeignet ist.

Aufgrund der geschilderten grundlagenwissenschaftlichen Entdeckungen ergeben sich folgende Anforderungen an das komplexe Wirkprofil geeigneter Pharmaka, die umfassend nur durch geeignete Wirkstoffkombinationen erzielt werden können:
a) Relaxation des venolären Endothels bzw. Inhibition der Kontraktilität der venolären Endothelzellen und
b) Inhibition der Produktion und Freisetzung ödemverursachender Eikosanoide aus gleichzeitig aktivierten, metabolisch kooperierenden Thrombozyten und Leukozyten.

Diese Aufgaben werden erfindungsgemäß durch eine Wirkstoffkombination gelöst, die mindestens einen Hemmstoff der - durch aktivierte: Leukozyten und Thrombozyten induzierbaren - Kontraktilität venolärer Endothelzellen und mindestens einen Hemmstoff der Cyclooxygenase 1, vorzugsweise mindestens ein nichtsteroidales Antiphlogistikum enthält.

Die erfindungsgemäße Wirkstoffkombination enthält in pharmakologisch verträglicher Dosierung mindestens eine Benzopyron-Verbindung als Hemmstoff der Kontraktilität venolärer Endothelzellen. Ausgenommen sind jedoch blutgerinnungshemmende Benzopyron-Verbindungen wie Dicumarol.

Solche in erfindungsgemäßen Wirkstoffkombinationen enthaltenen Benzopyrone leiten sich bevorzugt ab von α-Pyron- und γ-Pyron-Verbindungen der Grundformeln 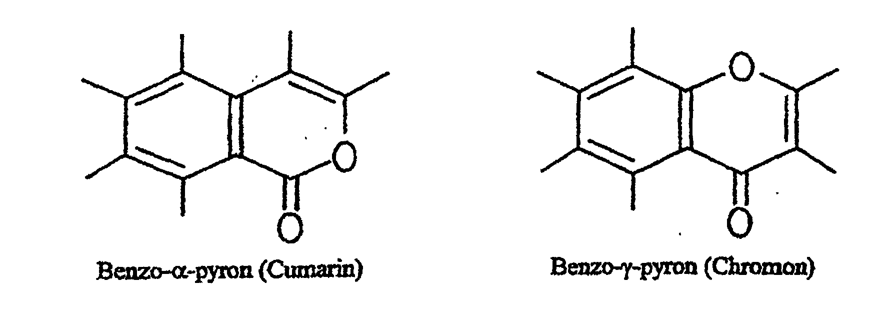 die am α-Pyron bzw. γ-Pyron-Grundgerüst einen oder mehrere Substituenten haben können, bei denen es sich bevorzugt um Hydroxylgruppen handelt. Diese Hydroxylgruppen können frei oder alkyliert bzw. hydroxyalkyliert sein, oder aber auch in Form von Glykosidderivaten vorliegen. Die Alkylreste sind vorzugsweise Methyl- oder Ethylgruppen. Die Glykosidbindung erfolgt bevorzugt in Position 3, üblicherweise mit Zuckern wie Rhamnose, Glucose, Glucorhamnose, Galaktose, Mannose, Lignan oder Arabinose. Dabei kommen alle optischen Enantiomeren in Frage.

Erfindungsgemäß bevorzugt verwendete γ-Pyronderivate sind in der Position 2 oder 3 phenyliert. Dadurch leiten sich die jeweils 3 Ringe enthaltenden Klassen der Flavone bzw. Isoflavone ab (Ringe A, B, C).

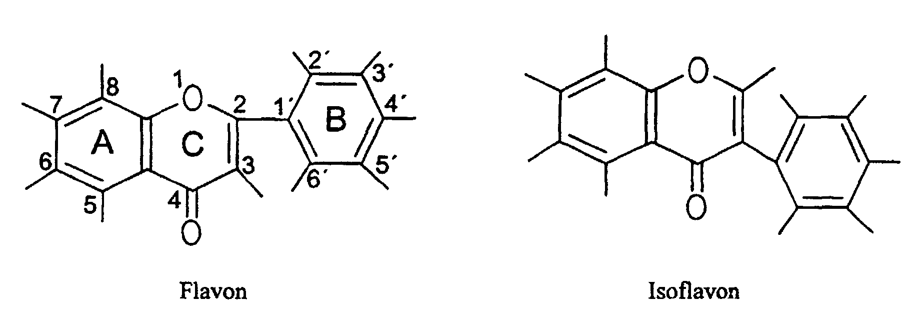

Durch Reduktion der 2-3 Bindung leiten sich die entsprechenden Flavane bzw. Isoflavane ab. Flavonole bzw. Isoflavonole unterscheiden sich von den Flavonen bzw. Isoflavonen durch Einführung einer zusätzlichen, häufig glykosylierten Hydroxylgruppe in Position 3. Erfindungsgemäße Anwendung können auch die Anthocyanidine beanspruchen, bei denen Ring C geöffnet ist.

Bevorzugt verwendete Flavonoidverbindungen haben eine oder mehrere Hydroxylfunktionen in den Ringpositionen 3, 5, 7, 2', 3', 4' und/oder 5', die auch in Form ihrer alkylierten bzw. hydroxyalkylierten Derivate vorliegen können. Die folgende Tabelle 1 benennt einige wichtige, natürlich vorkommende Vertreter dieser Verbindungen. In den Beispielen sind bevorzugte Benzopyron-Verbindungen aufgeführt.

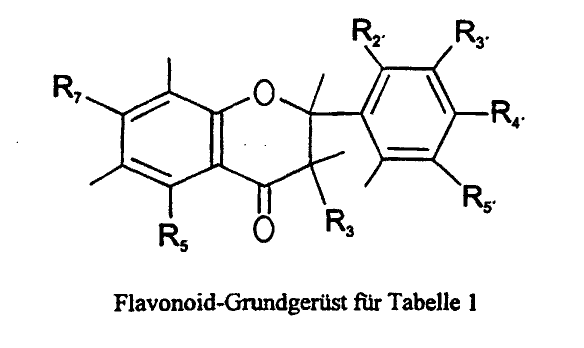

**Tabelle 1:**

| Natürlich vorkommende Flavonoide | | | | | | | | |
|---|---|---|---|---|---|---|---|---|
| **Name** | **Klasse** | **R**_{**3**} | **R**_{**5**} | **R**_{**7**} | **R**_{**2'**} | **R**_{**3'**} | **R**_{**4'**} | **R**_{**5'**} |
| Quercetin | Flavonol | OH | OH | OH | H | OH | H | H |
| Quercitrin | Flavonol | O-rh | OH | OH | H | OH | OH | H |
| Myricitrin | Flavonol | O-rh | OH | OH | H | OH | OH | H |
| Luteolin | Flavon | H | OH | OH | H | OH | OH | H |
| Kaempherol | Flavonol | OH | OH | OH | H | H | OH | H |
| Apigenin | Flavon | H | OH | OH | H | H | OH | H |
| Rutin | Flavonol | O-rh | OH | OH | H | OH | OH | H |
| Hesperitin | Flavonol | H | OH | OH | H | OH | O-me | H |
| Eriodictyol | Flavonol | H | OH | OH | H | OH | OH | H |
| Hesperidin | Flavonol | H | OH | O-rut | H | OH | O-me | H |
| Chrysin | Flavon | H | OH | OH | H | H | H | H |
| Techtochrysin | Flavon | H | OH | O-me | H | H | H | H |
| Silybin | Flavonol | OH | OH | OH | H | H | -O-lign-O | |
| Morin | Flavonol | OH | OH | OH | OH | H | OH | H |
| Naringenin | Flavonon | H | OH | OH | H | H | OH | H |
| Taxifolin | Flavonol | OH | OH | OH | H | OH | OH | H |
| Pinocembrin | Flavonol | H | OH | OH | H | H | H | H |
| Galangin | Flavonol | OH | OH | OH | H | H | H | H |
| Robinin | Flavonol | O-gal-rh | OH | OH | H | H | OH | H |
| Diosmetin | Flavon | H | OH | OH | H | OH | O-me | H |
| Kaempferide | Flavonol | OH | OH | OH | H | H | O-me | H |
| Fisetin | Flavonol | OH | H | OH | H | OH | OH | H |
| Rhamnetin | Flavonol | OH | OH | O-me | H | OH | OH | H |
| rh = Rhamnpside, gal = Galactose, lign = Lignan, rut = Rutosid, me = Methyl, gluc = Glucose | | | | | | | | |

Viele dieser Verbindungen kommen als Bestandteile von Nahrungsmitteln vor. Sie gelten hinsichtlich ihrer Toxizität und Nebenwirkungen als unbedenklich (Lit. 7c).

Erfindungsgemäß verwendbare nichtsteroidale Antiphlogistika umfassen alle selektiven Hemmstoffe der Cyclooxygenase 1. Spezielle Beispiele finden sich z.B. in der EP-B-0311677.

Bevorzugte, erfindungsgemäß verwendbare nichtsteroidale Antiphlogistika umfassen Verbindungen aus der Gruppe Acetylsalicylsäure, Arylpropionsäurederivate (z. B. Ibuprofen, Flurbiprofen und Naproxen), Arylessigsäurederivate (z. B. Diclofenac), Indolessigsäurederivate (z. B. Indometacin), Anthranilsäurederivate (z. B. Mefenaminsäure, Flufenaminsäure), Oxicame (z. B. Piroxicam und Tenoxicam) und Pyrazolidindione (z. B. Phenylbutazon).

Das erfindungsgemäß besonders bevorzugte nichtsteroidale Antiphlogstikum für die Wirkstoffkombination ist die Acetylsalicylsäure aufgrund ihrer geringen Plasmahalbwertszeit (ca. 25 Minuten) und ihrer hohen Selektivität und guten Verträglichkeit bei niedriger Dosierung.

Die erfindungsgemäße Wirkstoffkombination interveniert mit den oben geschilderten Pathomechanismen während Reperfusion ischämischer Organe und verhindern die entsprechenden Schäden kausal durch a) Protektion gegenüber oxidativen Gewebeschäden, b) Verhinderung interstitieller Ödeme durch Abdichtung des Gefäßendothels und c) gleichzeitige Prophylaxe einer Plättchen-, Leukozyten- und Endothelzellaktivierung. Optimierte hyperkaliämische Grundlösungen zur Aufnahme dieser Wirkstoffe verringern gleichzeitig das Energiedefizit der Organgewebe während der vorangehenden Ischämie.

Aus diversen Publikationen war zwar bekannt, daß Flavonoide in tierischen Membranen einlagern können und nach oraler Applikation im vaskulären Endothel akkumulieren (vgl. Lit. 8). Aus solchen Gründen wurde ihnen schon frühzeitig eine Ödem-präventive Wirkung im Rahmen der Therapie chronisch venöser Insuffizienzen im Beinbereich zugesprochen, die aber abzielte auf die Behandlung hydrostatisch (also nicht entzündlich) bedingter Beinödeme. Dieselben Wirkstoffe können im Tiermodell schmerzlindernd und fiebersenkend wirken, Einflüsse, die durch eine Cyclooxygenase- und Lipidoxygenase-hemmende Wirkung begründet sein sollen. Auch diverse hydrolytische (lysosomale) Enzyme werden bekanntlich durch Flavonoide stark gehemmt. Allergien oder Hypersensibilitätsreaktionen vom verzögerten Typ werden auch heute noch vielfach mit Dinatriumcromoglykat therapiert, einer Verbindung, die - wie die Flavonoide - ein Benzopyron-Ringsystem aufweist (Lit. 9). Die Wirkung der Flavonoide auf die im Rahmen allergischer Krankheitsmechanismen involvierten Mastzellen wurde auf die Inhibition einer ATPase in der Membran der Mastzellgranula zurückgeführt (Lit. 7c). Flavonoide bilden außerdem starke Komplexe mit zweiwertigen Schwermetallionen (Fe2+, Cu2+, Zn2+) und können leicht oxidiert werden (Lit. 7c). Diese Eigenschaften machen diese Verbindungen zu hochwirksamen Radikalfängern und Antoxidantien gerade in hochaktiven tierischen Geweben wie dem Gefäßendothel. Die in Rotwein in hoher Konzentration enthaltenen, und damit von der südfranzösischen Bevölkerung stark konsumierten Flavonoide sollen durch aggressive Radikale bedingte, ischämische Herzkrankheiten stark verringern können. Eine Präparation von Flavonoiden (90% Diosmin und 10% Hesperidin) kann einer neueren Arbeit nach die Leukozyten-Adhäsivität signifikant supprimieren, und es wurde spekuliert, daß diese Substanzen deshalb indirekt auch den Verlauf des Ischämie/Reperfusions-Syndroms abmildern könnten (Friesenecker B, Tsai AG, Intaglietta M: Oral administration of purified micronized flavonoid fraction suppresses leukocyte adhesion in ischemia-reperfusion injury: in vivo observations in the hamster skin fold. Int. J. Microcirc. Clin. Exp. (1994) 14:50-55).

Einer Flut von Publikationen war auch bereits zu entnehmen, daß nichtsteroidale Antiphlogistika wie Acetylsalicylsäure, Indometacin, Ibuprofen, Naproxen, Meclofenamat, Sulindac, Flufenamat ihre antiphlogistische Wirkung (Schmerzlinderung, Fiebersenkung) durch irreversible (Acetylsalicylsäure) bzw. kompetitive (alle anderen) Hemmung der Cyclooxygenase 1 entfalten. Dadurch stellen diese Wirkstoffe auch starke Hemmstoffe der Plättchen dar. Oral nach einer initialen, einmaligen Tagesdosis von 100 mg an den folgenden Tagen nur noch in Konzentrationen von 20-40 mg/d gegebene Acetylsalicylsäure konnte in einer breit angelegten klinischen Studie (an 17187 Patienten) ischämische Herzschäden signifikant - es wurde postuliert, durch Thrombozytenaggregationshemmung - verringern. Die Basis dafür ist eine irreversible Hemmung der Cyclooxygenase der Plättchen durch Acetylierung eines Serinrestes (Ser 530) dieses Enzyms durch Acetylsalicylsäure. Schon in einer Konzentration von 100 µM führt Acetylsalicylsäure bei 37°C innerhalb von 30 min zur Inaktivierung der Hälfte des aktiven Enzyms, nach 60 min zur kompletten Inaktivierung (Van der Oudera FJ, Buytenhek M, Nugteren DH, van Dorp DA: Acetylation of prostaglandin endoperoxide synthetase with acetylic acid. Eur. J. Biochem. (1980) 109:1-8; Mizuno K, Yamamoto S, Lands WEM: Effects of non-steroidal anti-inflammatory drugs on fatty acid cyclooxigenase and prostaglandin hydroperoxidase aktivities. Prostaglandins (1982) 23:743-757; De Witt DL, Smith NL: Primary structure of prostaglandin G/H synthetase from sheep vesicular gland determined from complementary DNA sequence. Proc. Nat. Acad. Sci. USA (1988) 85:1412-1416; Smith WL, Marnett LJ, De Witt DL: Prostaglandin and thromboxane biosynthesis. Pharmac. Ther. (1991)49:153-147).

Die kombinierte Applikation einer Benzopyron-Verbindung (z. B. eines Flavonoids) mit einem nichtsteroidalen Antiphlogistikum (z. B. Acetylsalicylsäure) ist bisher nur im Zusammenhang mit der Prävention einer Ischämie durch Thromboembolie für sinnvoll erachtet worden. In solchen Zusammenhängen wurden speziell Benzopyronderivate mit antikoagulatorischer Wirkung wie z.B. Cumarin-Derivate in Kombination mit Acetylsalicylsäure eingesetzt (Meschengieser SS, Fondevilla CG, Frontroth J, Santarelli MT, Lazzari MA: Low-intensity oral anticoagulation plus low-dose aspirin versus high-intensity oral anticoagulation alone: a randomized trial in patients with mechanical prosthetic heart valves. J. Thorac. Cardiovasc. Surg. (1997) 113:910-916). Auch die Kombination von Flavonoiden mit Acetylsalicylsäure zur Verhinderung von Thrombosen im Tierversuch ist bekannt (Hladovic J: Antithrombotic effects of some flavonoids alone and combined with acetylsalicylic acid. Arzneim.-Forsch. / Drug Res. (1977) 27:1989-1992). Derartige Applikationen und Therapieansätze zielen aber klarermaßen lediglich auf die Verhinderung von Thrombosen ab, die nur einer der Gründe für Ischämie in versorgungsabhängigen Gefäßstrecken ist. Das pathogenetisch sich in der Folge ischämischer Prozesse stets erst im Augenblick der Reperfusion ergebende Reperfusionssyndrom wurde in derartigen Studien niemals angesprochen bzw. untersucht.

Ein Hinweis auf Wirkstoffgemische, Infusionslösungen oder Inhalate zur Prophylaxe und Therapie von u.a. ischämischen Organschäden und Reperfusionssyndromen gemäß der vorliegenden Erfindung läßt sich aus den bisherigen therapeutischen Anwendungen der Wirkstoffklassen nicht entnehmen, letzteres auch umso mehr, als die hier abgeleiteten und kausal mit dieser Wirkstoffkombination therapierbaren Pathomechanismen ischämisch bedingter Perfusionsschäden zum Zeitpunkt der Anmeldung noch unbekannt waren. Der vorliegenden Erfindung liegt die unerwartete Entdeckung zugrunde, daß die Endothelzellen der kleinsten Venen (venoläre Endothelzellen) sich unter dem Einfluß aktivierter Blutzellen, insbesondere von Leukozyten und Thrombozyten, kontrahieren können, daß Benzopyron-Verbindungen, wie z.B. Flavonoide, spezifische Hemmwirkungen auf die Kontraktilität der venolären Endothelzellen entfalten und durch ihre antioxidativen Eigenschaften die aus aktivierten Leukozyten freigesetzten Oxidantien neutralisieren können, und daß nichtsteroidale Antiphlogistika wie z.B. Acetylsalicylsäure die Thrombozyten hemmen und dadurch die Anlieferung von Eikosanoiden unterbinden, die in Leukozyten zu Wirkstoffen verwandelt werden können, die wiederum die Kontraktilität venolärer Endothelzellen induzieren. Diese Entdeckung ermöglicht die Bereitstellung der erfindungsgemäßen Wirkstoffkombination zur gezielten Protektion des venolären Endothels und damit zur Prophylaxe und Therapie von ischämischen Organschäden und Reperfusionssyndromen. Der Nachweis der Wirkung von Verbindungen als Hemmstoffe der Kontraktilität von venolären Endothelzellen kann dabei z. B. nach der in Beispiel 12 beschriebenen Versuchsmethodik erfolgen.

Die folgenden experimentellen Untersuchungen demonstrieren die Wirkung am Beispiel der Acetylsalicylsäure in Kombination mit bestimmten Flavonoiden an Hand dreier Versuchsgruppen. Dabei wird jeweils mit verschiedenen experimentellen Systemen der Einfluß von Granulozyten (PMN) und/oder Plättchen (Thrombozyten) aufvenoläres Endothel untersucht.

### Versuchsgruppe 1

Pathogene Wirkung von gleichzeitig aktivierten PMN und Thrombozyten im ischämischen Herzen.

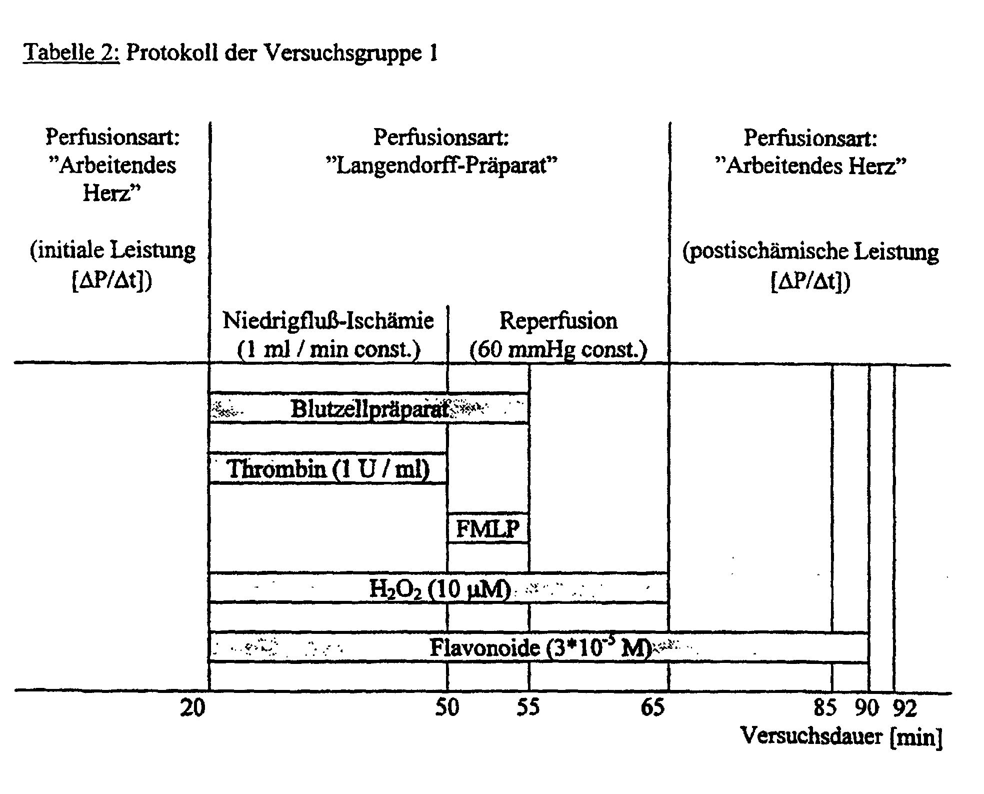

Als Studienobjekt dienten arbeitende, also Volumen gegen bestimmte Drucke auswerfende Meerschweinchenherzen. Gemessen wurde dabei jeweils die Druckanstiegsgeschwindigkeit (ΔP/Δt) als Ausdruck der Myokardkontraktilität, der interstitielle Wassergehalt (µl/g) als Ausdruck der Bildung interstitieller Ödeme und der Koronarfluß als Ausdruck der koronaren Durchblutungsregulationsfähigkeit.

Tabelle 2 zeigt ein typisches experimentelles Protokoll zur Messung des Einflusses verschiedener Blutzellpräparateⁱ (Granulozyten = PMN, Thrombozyten = T) auf diese Zustandsparameter der Herzen. Thrombin, FMLP (ein Entzündungspeptid; chemische Bezeichnung N-Formyl-Methionyl-Leucyl-Phenylalanin) und Wasserstoffperoxid induzieren eine Interaktion der Blutzellen mit dem koronaren Endothel bei diesen Versuchen.

**Tabelle 3:**

| **Eingesetztes Flavonderivat (Konzentration 3*10**^{**-5**} **M)** | **Herzleistung [% der initialen Leistung]** | | **Interstitielles Volumen [µl / g]** | | **Koronarfluß [% des Basalflusses]** | |
|---|---|---|---|---|---|---|
| | **PMN +T**_{**ASS**} | **PMN+T** | **PMN+T**_{**ASS**} | **PMN+T** | **PMN+T**_{**ASS**} | **PMN+T** |
| (Kontrolle) | 72, 81, 76, 85, 82, 70 | 35, 46, 28, 33, 37, 18, 31 | 133, 124, 127, 135, 141, 117 | 350, 370, 310, 290, 293, 312 | 88, 82, 79, 83, 77, 87 | 38, 15, 19, 26, 36 |
| Quercetin | 82, 86, 90,85,94 | 66, 69, 58, 44, 70 | 108, 113, 107, 104, 125, 118 | 159, 178, 181, 185, 162 | 101,107, 89, 95, 91 | 61, 49, 49, 55, 62, 40 |
| Dihydroxyethylrutosid | 93, 94, 86, 84, 89 | 49, 58, 65, 72, 78, 74 | 98, 104, 95, 115, 107, 109 | 144, 186, 151, 157, 165 | 102, 107, 98, 93, 95, 89 | 49, 51, 39, 47, 64, 69 |
| Catechin | 75, 68, 67, 81, 81, 77 | 36, 39, 31, 26, 18 | 145, 157, 163, 148, 166 | 351, 310, 311, 317, 370 | 88, 78, 82, 79, 85 | 37, 27, 34, 17, 14, 28 |
| Apigenin | 94, 96, 89, 88, 97 | 52, 67, 74, 73, 64, 71 | 95, 120, 105, 102, 97, 91 | 142, 201, 177,166, 130 | 102, 107, 98, 95, 91, 109 | 56, 49, 56, 53, 59, 65 |
| Naringenin | 90, 97, 84, 94, 82, 89 | 63, 49, 54, 69, 68 | 98, 117, 107, 109, 102 | 169, 175, 189, 205, 198 | 107, 98, 93, 92, 89, 101 | 57, 55, 63, 51, 45, 46 |
| Flavon | 78, 81, 69, 82, 83 | 31, 31, 18, 35, 29 | 129, 153, 145, 148, 169 | 344, 389, 388, 358, 346 | 88, 77, 73, 81, 87 | 33, 19, 31, 28, 29 |
| 4-Chromanon | 79, 81, 74, 71, 69, 78 | 27, 29, 38, 31, 17, 22 | 155, 148, 168, 161, 165 | 373, 348, 354, 318, 356 | 88, 74, 79, 81, 82, 79 | 44, 19, 35, 26, 29 |

ⁱ Als Blutzellpräparate wurden eingesetzt:
a) hochreine PMN (1-2*10⁵/ml),
b) Thrombozyten (1-2*10⁶/ml), nach einstündiger Vorinkubation mit 100 µM Acetylsalicylsäure gewaschen (T_{ASS}) bzw. nicht mit Acetylsalicylsäure vorinkubiert (T),
c) Gemische von PMN und T_{ASS} (bzw. T) in jeweils analogen Konzentrationen.

Tabelle 3 gibt die unter diesen Versuchsbedingungen bei Einsatz eines Gemisches von PMN und Thrombozyten (T_{ASS} = nach einstündiger Vorinkubation mit 100µM Acetylsalicylsäure gewaschene Thrombozyten, T = nicht mit Acetylsalicylsäure vorinkubierte Thrombozyten) erhaltenen Meßwerte wieder.

### Hauptergebnisse der Versuchsgruppe 1:

Alle postischämischen Funktionsdaten (ΔP/Δt; interstitielles Volumen; Koronarfluß) weisen gegenüber den entsprechenden initialen (vor Ischämie gemessenen) Werten eine deutliche Verschlechterung auf, die durch die akute Applikation von bestimmten Flavonoiden (Quercetin, Dihydroxyethylrutosid, Apigenin, Naringenin, chemische Formeln siehe Tabelle 1) abgeschwächt, aber selbst in hohen Konzentrationen nicht vollständig aufgehoben werden kann. Ebenso führt die Vorinkubation der Thrombozyten mit Acetylsalicylsäure in einer Konzentration von 100 µM, durch die Cyclooxygenase der Thrombozyten vollständig und irreversibel inhibiert wird, zu einer lediglich teilweisen Abschwächung der Verschlechterung der postischämischen Funktionsdaten. Nur durch die kombinierte Applikation der genannten Flavonoide mit Acetylsalicylsäure kann die Verschlechterung der postischämischen Funktionsdaten vollständig aufgehoben werden.

Überraschenderweise hat die kombinierte Applikation von Acetylsalicylsäure mit den oben genannten Flavonoiden also einen hochgradig protektiven Effekt auf das ischämische Herz.

Daneben geht aus den Versuchsergebnissen hervor, daß die Benzopyron-Derivate Catechin, Flavon und 4-Chromanon offenbar keine protektive Wirkung entfalten.

### Versuchsgruppe 2

Als Studienobjekt dienten bis zur Konfluenz etablierte Schichten venolärer Endothelzellen auf porösen Filtern, die in einer speziell entwickelten Apparatur zur Trennung zweier Flüssigkeitskompartimente eingesetzt wurden. So konnte z. B. der druckabhängige Wassertransport (hydraulische Konduktivität = L_{P} mit der Dimension 10⁻⁵ cm / s / cm H₂O) über die gezüchteten Endothelschichten unter dem Einfluß von Blutzellen unter entzündlichen Bedingungen studiert werden.

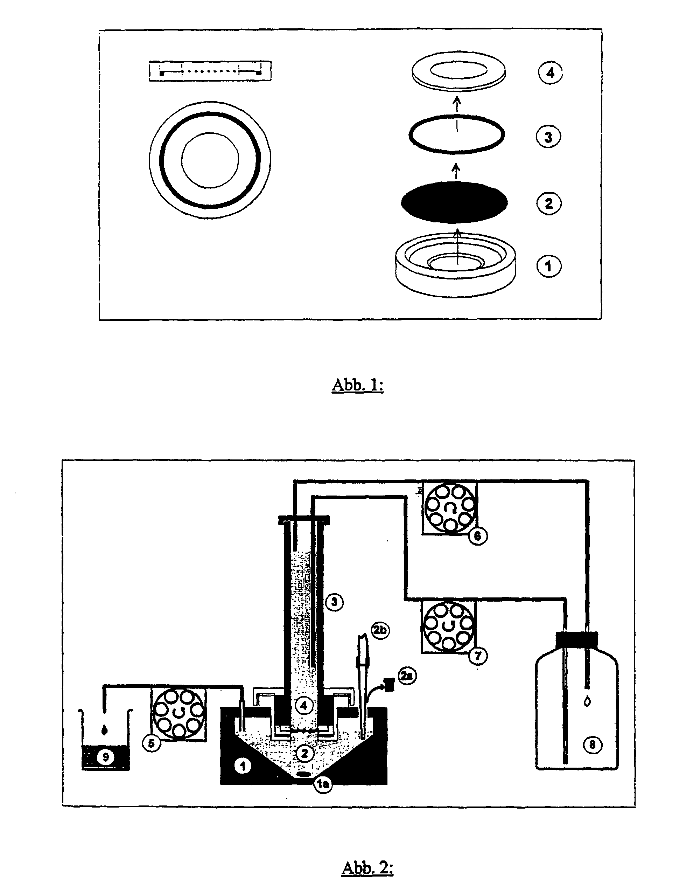

Details der Versuchsanordnung gehen aus den Abb. 1 und 2 hervor.
- Abbildung 1: zeigt ein wiederverwendbares, autoklavierbares Filterspannsystem bestehend aus (1) dem Formteil aus Polycarbonat, (2) dem Polycarbonatfilter, (3) dem stählernen Spannring und (4) dem Haltering aus Polycarbonat. Die Darstellung rechts neben dem Stapel der Einzelteile zeigt einen Querschnitt und eine Aufsicht des zusammengefügten Systems.
- Abbildung 2: zeigt eine Versuchsapparatur zur Messung hydraulischer Konduktivitäten von konfluenten Zellschichten, (1) Basisteil mit Magnetrührer (1a), (2) basales Flüssigkeitskompartiment mit Stopfen (2a) bzw. Zugang für Eppendorfpipetten (2b), (3) Steigrohr, (4) oberes Flüssigkeitskompartiment, zwischen den Flüssigkeitskompartimenten (2) und (4) liegt der ausgespannte Filter, (5), (6), (7) peristaltische Pumpen, (8) Vorratsgefäß für die Konstanthaltung des hydrostatischen Niveaus im oberen Flüssigkeitskompartiment, (9) Sammelgefäß für filtriertes Volumen.

Die folgende Tabelle 4 zeigt einige exemplarische Versuchsdaten.

**Tabelle 4:**

| **Nr.** | **Inkubationsansätze** | **L**_{**P**} **[10**^{**-5**} **cm/s/cm H**_{**2**}**O]** |
|---|---|---|
| 1 | Isotonische Plasmasalzlösung mit Zusatz von 0,1% Albumin (IPA) | 1,80 ± 0,03 |
| 2 | IPA + Thrombin (1 U/ml) | 1,70 ± 0,12 |
| 3 | IPA + Thrombozyten (10⁶/ml) + Thrombin (1 U/ml) | 1,58 ± 0,07 |
| 4 | IPA + PMN (10⁵/ml) + Thrombin (1 U/ml) + 1 µM FMLP | 5,40 ± 0,50 |
| 5 | IPA + PMN (10⁵/ml) + Thrombin (1 U/ml) + 1 µM FMLP + 50 µM Apigenin | 1,75 ± 0,06 |
| 6 | IPA + PMN (10⁵/ml) + Thrombin (1 U/ml) + 1 µM FMLP + 50 µM Acetylsalicylsäure | 5,78 ±0,68 |
| 7 | IPA + PMN (10⁵/ml) + Thrombozyten (10⁶/ml) + Thrombin (1 U/ml) + 1 µM FMLP | 33,7 ± 3,3 |
| 8 | IPA + PMN (10⁵/ml) + Thrombozyten (10⁶/ml) + Thrombin (1 U/ml) + 1 µM FMLP + 50 µM Apigenin | 25,5 ± 4,1 |
| 9 | IPA + PMN (10⁵/ml) + Thrombozyten (10⁶/ml) + Thrombin (1U/ml) + 1 µM FMLP + 50 µM Apigenin + 50 µM Acetylsalicylsäure | 2,10 ± 0,08 |
| 10 | IPA + Überstand eines Gemenges aus mit Thrombin (1 U/ml) aktivierten Thrombozyten (10⁶/ml) und mit 1 µM FMLP aktivierten PMN (10⁵/ml) | 31,2 ± 3,1 |
| 11 | IPA + Überstand eines Gemenges aus mit Thrombin (1 U/ml) aktivierten Thrombozyten (10⁶/ml) und mit 1 µM FMLP aktivierten PMN (10⁵/ml) + 50 µM Apigenin | 1,90 ± 0,07 |
| 12 | IPA + Überstand eines Gemenges aus mit Thrombin (1 U/ml) aktivierten Thrombozyten (10⁶/ml) und mit 1 µM FMLP aktivierten PMN (10⁵/ml) + 50 µM Acetylsalicylsäure | 28,2 ± 1,7 |

### Ergebnisse:

Thrombin und / oder Thrombozyten haben keinen Einfluß auf L_{P} (Nr. 1,2 und 3).

Selektiv applizierte, mit FMLP aktivierte PMN erhöhen die L_{P} um ca. 300 % (Nr. 4), wahrscheinlich durch oxidative Schädigung des Endothels, Apigenin schützt (Nr. 5) aufgrund seiner anitoxidativen Wirkung (Interzellularspalten bleiben geschlossen, keine Erhöhung von L_{P}), Acetylsalicylsäure hat selbst in einer Konzentration von 50 µM keinen Effekt (Nr. 6).

Gleichzeitig aktivierte Thrombozyten und PMN sezernieren Endothel-konstriktive Substanzen, die zu einer ca. 1600 %igen Erhöhung von L_{P} führen (Nr. 7). Rasterelektronenmikroskopische Studien zeigten, daß nun die Interzellularspalten weit geöffnet werden (vgl. Experimente der Versuchsgruppe 3). Apigenin allein hemmt diesen Effekt nur schwach (Nr. 8). Apigenin und Acetylsalicylsäure in Kombination unterbinden diesen Plättchen- und Granulozyten-induzierten Mechanismus überraschenderweise vollständig (Nr. 9). Der Vergleich der Versuchsergebnisse Nr. 8 und Nr. 9 weist somit darauf hin, daß Apigenin und Acetylsalicylsäure in Kombination eine überadditive Wirksamkeit im Sinne einer synergistischen Wirkungsverstärkung entfalten.

### Versuchsgruppe 3

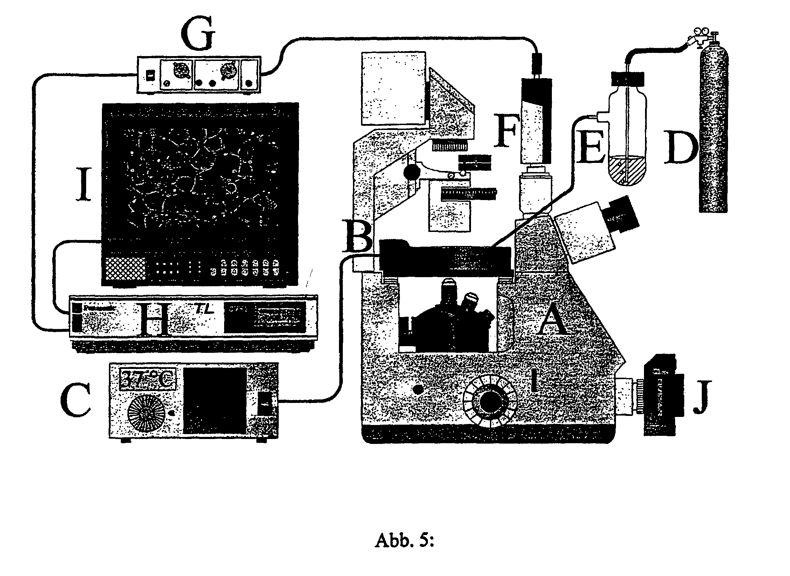

In vitro kultivierte Schichten aus venolären Endothelzellen (VEC) werden unter Einsatz von Zeitraffervideomikrokinematographie (siehe Abb. 5) mit speziell entwickelter Methodik direkt beobachtet. Die durch aktivierte Thrombozyten (Anwendung von 1 U/ml Thrombin) und aktivierte PMN (Anwendung von 1 µM FMLP) induzierte Öffnung von Endothelbarrieren (= Öffnung der Interzellularspalten der Endothelzellen) kann so im Zeitablauf dokumentiert werden. Als Beispiel dient eine Serie von Aufnahmen, die den direkten Angriff der Blutzellen dokumentiert (Abb. 3) bzw. eine analoge Serie, die mit dem Überstand der aktivierten Blutzellen durchgeführt wurde (Abb. 4).

### Erläuterungen zu Abb. 3:

a) Ausgangskultur (dichter Zellrasen), b) 8 min nach Zugabe aktivierter Granulozyten (=PMN) und Thrombozyten, c) wie b, aber nach 11 min, d) 17 min später, unmittelbar vor Zugabe der Wirkstoffe Trihydroxyethylrutosid und Acetylsalicylsäure (je 50 µM), e) 15 min nach Wirkstoffzusatz, f) nach weiteren 18 min, g) nach weiteren 22 min, h) nach weiteren 42 min. Die durch die Blutzellen zunächst geweiteten Interzellularspalten werden durch die Wirkstoffe wieder abgedichtet.

Flavonoide (wie z. B. Trihydroxyethylrutosid) und Acetylsalicylsäure in Kombination schützen also auch in Anwesenheit aktivierter Blutzellen gegen entzündliche Ödeme.

Die alleinige Applikation von Flavonoiden wie Trihydroxyethylrutosid (50 µM) oder von Acetylsalicylsäure (50 µM) führt in Anwesenheit der aktivierten Blutzellen jedoch nicht zur Abdichtung der Interzellularspalten.

### Erläuterungen zu Abb. 4:

a) Ausgangskultur (dichter Zellrasen), b) 15 min nach Zugabe einer sehr geringen Menge Überstand aktivierter PMN und Thrombozyten (die vorher abzentrifugiert wurden), dann Zugabe einer größeren Menge Überstand, c) 3 min später deutliche Öffnung der Spalten), d) weitere 3 min später (extreme Spaltenöffnung), anschließend Zugabe von Trihydroxyethylrutosid (ohne Acetylsalicylsäure), e) 9 min später bereits deutlich werdende Abdichtung der Spalten, f) weitere 6 min später, g) weitere 10 min später, h) weitere 48 min später. Zu diesem Zeitpunkt läßt sich unter dem Einfluß der Flavonoidverbindung bereits eine fast vollständige Reparatur der Endothelschicht konstatieren.

In Abwesenheit von Blutzellen haben Flavonoide wie Trihydroxyethylrutosid schon bei alleiniger Applikation einen Endothel-reparativen Effekt.

### Erläuterungen zu Abb. 5:

- Abbildung 5: zeigt den experimentellen Aufbau für die videomikroskopische Beobachtung zellulärer Interaktionen an Endotheloberflächen: A-Gewebekulturmikroskop, B-Spezialinkubator mit Steuereinheit C, D-Gaszylinder mit 5% CO₂, E-Waschflasche, F-Videokamera, G-Kamera-Kontroll-Einheit, H-Videorecorder, I-Monitor, J-Fotoapparat.

Zur Optimierung auf die jeweilige organ- bzw. indikationsspezifische Anwendung können dem erfindungsgemäßen Wirkstoffgemisch z. B. die folgenden Verbindungen zugesetzt werden:
a) Antoxidantien wie z. B. Ascorbinsäure, Harnsäure oder Vitamin E
   Diese Verbindungen wirken als Schutz gegen die im Metabolismus vor allem während und nach Ischämie gebildeten aggressiven Sauerstoffverbindungen (z. B. Wasserstoffperoxid, Sauerstoffradikaie, hypochlorige Säure).
b) Inosin
   Inosin dient als Vorläufersubstanz der zellulären Adeninnukleotid- und Hamsäuresynthese.
c) Asparagin- und Glutaminsäure
   Diese Aminosäuren fördern den Energiestoffwechsel besonders nach Ischämie.
d) Arginin
   Die essentielle Aminosäure Arginin dient als Quelle für endotheliales Stickoxid.

In allen Fällen, in denen die genannten Wirkstoffe in Form von Lösungen appliziert werden, können weiterhin enthalten sein:
e) Plasmaexpander, hydrophile Kolloide, Dextrane, Hydroxyethylstärke, Albumin
   Diese Inhaltsstoffe dienen dem Aufbau eines ausreichenden onkotischen Druckes im Intravasalraum.
f) Hämoglobinersatzstoffe wie z. B. Fluorkohlenwasserstoffe
   Diese dienen der Erhöhung der Sauerstoffkonzentration im Intravasalraum.

Weitere Inhaltsstoffe umfassen Nährsubstrate wie Glucose, Insulin, Pyruvat, Ribose; zusätzliche Aminosäuren; Vitamine; Fette wie z. B. Phosphatide, insbesondere Lecithin; sowie Stabilisatoren wie z. B. Tokopherole.

Die bei allen diesen Zusatzstoffen angestrebten Konzentrationen richten sich nach der jeweiligen Indikation (vgl. Beispiele 1 bis 11).

Wenn die Wirkstoffkombination als Lösung verabreicht wird, kommen als Lösungsmittel alle isotonischen, normo- bzw. hyperkaliämischen, klinisch verwendeten Salzlösungen in Frage. Vorzugsweise wird dabei endotoxinfreies Wasser ("high quality water") verwendet. Die Pufferung erfolgt vorzugsweise im pH-Bereich 7,2-7,6 unter Verwendung einer pharmakologisch verträglichen Puffersubstanz. Eine bevorzugte Puffersubstanz ist Histidin.

Vorzugsweise wird das in gepufferter Lösung zubereitete Wirkstoffgemisch als Lyophilisat konserviert und kurz vor Gebrauch in einer entsprechenden Menge von endotoxinfreiem Wasser rekonstituiert.

Für die Herstellung aller im folgenden beschriebenen Lyophilisate wird folgendermaßen verfahren:

Das Gemenge der Komponenten wird in der gewünschten Menge an endotoxinfreiem Wasser gelöst und vor dem Lyophilisieren auf pH 7,4 eingestellt. Das Lyophilisat ist lichtgeschützt aufzubewahren.

Die Sterilisation aller genannten Lösungen erfolgt in bekannter Weise durch Sterilfiltration (Porengröße 0,22 µm).

Die Beispiele erläutern die Erfindung. Sie sind nicht beschränkend aufzufassen.

In allen folgenden Beispielen für phannakologische Aufbereitungen des Wirkstoffgemisches mit Ausnahme von Beispiel 10 wird das klinisch gut bekannte und definiert isolierbare Trihydroxyethylrutosid verwendet. Alternativ dazu kann beispielsweise jedoch auch jedes der in Tabelle 1 aufgeführten 23 Flavonoide in der für Trihydroxyethylrutosid angegebenen Konzentration eingesetzt werden. Therapeutisch sinnvoll erscheint darüber hinaus der gesamte Konzentrationsbereich (in der verabreichten Perfusions- bzw. Infusionslösung) von 10 µM bis 250 µM, sowohl für das verabreichte Flavonoid wie auch für die Acetylsalicylsäure.

### Beispiel 1

Herstellung von 1000 ml einer kristalloiden Lösung zur Verwendung als kardioplegische Infusionslösung zur Erzielung des Herzstillstandes im Rahmen herzchirurgischer Operationen und als Organpräservationslösung im Rahmen von Organtransplantationen zur Verlängerung der Überlebenszeit entnommener Organe und zur Prophylaxe von Abstoßungsreaktionen:

| | | | |
|---|---|---|---|
| a) | Lyophilisat: | Trihydroxyethylrutosid | 78 mg |
| | | Acetylsalicylsäure | 18 mg |
| | | Ascorbinsäure | 18 mg |
| | | Harnsäure | 17 mg |
| | | Inosin | 27 mg |
| | | Asparaginsäure | 13,3 mg |
| | | Glutaminsäure | 14,6 mg |
| | | Arginin | 17,4 mg |
| b) | Kardioplegische Grundlösung [mM] | | |
| | | NaCl | 15 |
| | | KCl | 9 |
| | | MgCl₂ | 4 |
| | | Glucose | 5 |
| | | Kaliumhydrogen-2-oxoglutarat | 1 |
| | | Histidin-HCl (H₂O) | 180 |
| | | Mannit | 30 |

### Beispiel 2

Herstellung eines Zusatzes zu Blutkardioplegischen Lösungen:

| | | | |
|---|---|---|---|
| a) | Lyophilisat: | Trihydroxyethylrutosid | 390 mg |
| | | Acetylsalicylsäure | 90 mg |
| | | Ascorbinsäure | 90 mg |
| | | Harnsäure | 17 mg |
| | | Inosin | 135 mg |
| | | Asparaginsäure | 66,5 mg |
| | | Glutaminsäure | 73 mg |
| | | Arginin | 87 mg |

Dieses Lyophilisat wird 1000 ml der herkömmlich verwendeten Blutkardioplegie-Stammlösung zugesetzt. Die Mischung wird danach mit Eigenblut des Patienten im Verhältnis 1 Teil Stammlösung zu 4 Teilen Patientenblut vermischt.

### Beispiel 3

Herstellung von 1000 ml einer Lösung zur präoperativen und intraoperativen Verabreichung als Volumenersatz und/oder Prophylaktikum bei chirurgischen Operationen (z. B. Thoraxoperationen, Herzoperationen, Bauchoperationen, sonstige größere Operationen) und interventionell-kardiologischen (z. B. bei PTCA, Stentimplantationen, PTA, während und nach der Auflösung von Blutgefäßverschlüssen durch frische oder ältere Gerinnsel bei Venenthrombosen und arteriellen Thrombosen, Lungenembolien und Wiedereröffnung von künstlichen arteriovenösen Fisteln) oder diagnostischen Eingriffen, die mit einer partiellen Ischämie einhergehen können, bei akuten Gefäßverschlüssen (z. B. beim akuten Myokardinfarkt, beim Schlaganfall, Thromboembolie) und zur Prophylaxe und Therapie von Abstoßungsreaktionen nach Organtransplantation:

| | | | |
|---|---|---|---|
| a) | Lyophilisat: | Trihydroxyethylrutosid | 78 mg |
| | | Acetylsalicylsäure | 18 mg |
| | | Ascorbinsäure | 18 mg |
| | | Arginin | 17,4mg |
| b) | Grundlösung: | | |
| | Als Grundlösung können alle klinisch gebräuchlichen und pH 7,4 gepufferten isotonischen Infusionslösungen zum Volumenersatz (1000 ml) verwendet werden. | | |

### Beispiel 4

Herstellung von 1000 ml einer Lösung zum Volumenersatz und zur Therapie der entstehenden Gewebsödeme bei Brandverletzungen:

| | | | |
|---|---|---|---|
| a) | Lyophilisat: | Trihydroxyethylrutosid | 78 mg |
| | | Acetylsalicylsäure | 18 mg |
| | | Ascorbinsäure | 18 mg |
| | | Asparaginsäure | 13,3 mg |
| | | Glutaminsäure | 14,6 mg |
| | | Arginin | 17,4mg |
| b) | Grundlösung: | | |
| | Als Grundlösung können alle klinisch gebräuchlichen und pH 7,4 gepufferten isotonischen Infusionslösungen zum Volumenersatz (1000 ml) verwendet werden. Diesen Lösungen können zusätzlich die klinisch gebräuchlichen hochmolekularen Verbindungen bzw. Plasmaexpander wie Hydroxyethylstärke, Dextrane, hydrophile Kolloide oder Albumin zugesetzt werden. | | |

### Beispiel 5

Herstellung von 1000 ml einer Lösung zur Organperfusion bei Eingriffen unter isolierter Perfusion eines Organes (z. B. isolierte Extremitätenperfusion, gefäßchirurgische Eingriffe):

| | | | |
|---|---|---|---|
| a) | Lyophilisat: | Trihydroxyethylrutosid | 78 mg |
| | | Acetylsalicylsäure | 18 mg |
| | | Ascorbinsäure | 18 mg |
| | | Harnsäure | 17 mg |
| | | Inosin | 27 mg |
| | | Asparaginsäure | 13,3 mg |
| | | Glutaminsäure | 14,6 mg |
| | | Arginin | 17,4 mg |
| b) | Grundlösung | | |
| | Als Grundlösung können alle klinisch gebräuchlichen und pH 7,4 gepufferten isotonischen Infusionslösungen zum Volumenersatz (1000 ml) verwendet werden. | | |

### Beispiel 6

Herstellung von 20 ml einer hochdosierten Lösung zur intravenösen Injektion als Sofortbe-handlung des Schocks oder beim Infarkt

| | | | |
|---|---|---|---|
| a) | Lyophilisat: | Trihydroxyethylrutosid | 250 mg |
| | | Acetylsalicylsäure | 60 mg |
| b) | Grundlösung: | | |
| | Isotonische Kochsalzlösung (20 ml) | | |

### Beispiel 7

Herstellung von 250 ml einer Lösung zur Infusion bei Organspendern vor Entnahme von Organen:

| | | | |
|---|---|---|---|
| a) | Lyophilisat: | Trihydroxyethylrutosid | 300 mg |
| | | Acetylsalicylsäure | 80 mg |
| | | Ascorbinsäure | 72 mg |
| | | Inosin | 50 mg |
| | | Asparaginsäure | 30 mg |
| | | Glutaminsäure | 30 mg |
| | | Arginin | 30 mg |
| b) | Grundlösung | | |
| | Als Grundlösung werden 250 ml einer klinisch gebräuchlichen, bei pH 7,4 gepufferten isotonischen Infusionslösung, z. B. isotonische Kochsalzlösung, verwendet. | | |

### Beispiel 8

Herstellung einer Tablette zur Prophylaxe und Langzeittherapie der Arteriosklerose, insbesondere der koronaren Herzerkrankung, und sonstiger Gefäßerkrankungen sowie zur Verabreichung nach Organtransplantation:

| | | | |
|---|---|---|---|
| a) | Wirkstoffe: | Trihydroxyethylrutosid | 100 mg |
| | | Naringenin | 100 mg |
| | | Quercetin | 100 mg |
| | | Apigenin | 100 mg |
| | | Acetylsalicylsäure | 50 mg |
| b) | Trägerstoffe | | |
| | Als Träger kann ein Gemisch üblicher Trägerstoffe wie z. B. Maisstärke, Hochdispergiertes Siliciumdioxid, Calciumcarboxymethylcellulose, Talkum und Magnesiumstearat verwendet werden. | | |

### Beispiel 9

Herstellung einer Tablette zur Dauertherapie von chronischen Entzündungen (z. B. Bindegewebserkrankungen, Vaskulitiden, Arteriitiden)

| | | | |
|---|---|---|---|
| a) | Wirkstoff: | Trihydroxyethylrutosid | 400 mg |
| | | Diclofenac | 50 mg |
| b) | Trägerstoffe | | |
| | Als Träger kann ein Gemisch üblicher Trägerstoffe wie z. B. Maisstärke, Hochdispergiertes Siliciumdioxid, Calciumcarboxymethylcellulose, Talkum und Magnesiumstearat verwendet werden. | | |

### Beispiel 10

Herstellung von 100 g Salbe/Creme/Paste zur Behandlung von Hauterscheinungen im Rahmen von Vaskulitiden, Verletzungen, Prellungen oder geringgradigen Verbrennungen:

| | | | |
|---|---|---|---|
| a) | Wirkstoffe: | Tetrahydroxyethylrutosid | 2000 mg |
| | | Diclofenac | 1000 mg |
| b) | Grundstoffe: | | |
| | Als Grundstoffe dienen die gebräuchlichen Grundzubereitungen für Salben, Cremes und Pasten. | | |

### Beispiel 11

Herstellung von 100 ml Tropfen zum Einmassieren in das Zahnfleisch zur Prophylaxe und Therapie von Parodontose:

| | | | |
|---|---|---|---|
| a) | Wirkstoffe: | Trihydroxyethylrutosid | 1000 mg |
| | | Acetylsalicylsäure | 10 mg |
| | | Ascorbinsätue | 1000 mg |
| | | Vitamin E | 50 mg |
| b) | Grundlösung | | |
| | Ethanol 50 Vol-%, endotoxinfreies Wasser 50 Vol-% | | |

### Beispiel 12

### Nachweis der Wirkung von Verbindungen als Hemmstoffe der Kontraktilität von venolären Endothelzellen auf der Grundlage der Versuchsanordnung von Versuchsgruppe 2

Der Nachweis der Wirkung von Verbindungen als Hemmstoffe der Kontraktilität von venolären Endothelzellen erfolgt analog zur in Versuchsgruppe 2 charakterisierten Vorgehensweise unter Verwendung der dort beschriebenen Versuchsanordnung (vgl. Abb. 1 und 2) und wird hier am Beispiel des Apigenins (chemische Formel siehe Tabelle 1) dargestellt.

Venoläre Endothelzellen werden unter Verwendung einer speziellen, nachstehend beschriebenen Technik isoliert und auf porösen Polycarbonatfiltern (Porendurchmesser 0,4 µm, Fa. Nucleopore, Pleasanton, USA) unter Verwendung ebenfalls nachstehend beschriebener Zellkulturtechniken bis zur Konfluenz kultiviert. Diese endothelbeschichteten Filter werden in einer speziellen Apparatur (siehe Abb. 1 und 2) zur Trennung zweier Flüssigkeitskompartimente eingesetzt. Als Meßgröße für die Kontraktilität der venolären Endothelzellen dient der druckabhängige Wassertransport (hydraulische Konduktivität = L_{P} mit der Dimension 10⁻⁵ cm / s / cm H₂O) über die gezüchteten Endothelschichten, der bei Kontraktion der Endothelzellen durch Übergang von der pflastersteinartigen zur sphärischen Form (vgl. Versuchsgruppe 3 mit Abb. 3 und 4) und die damit einhergehende Öffnung von Interzellularspalten zunimmt

Eine Lösung von Plasmasalzen (=physiologische Salzlösung, Zusammensetzung siehe unten) mit Zusatz von 0,1% Albumin und von aus peripherem Blut wie nachfolgend beschrieben isolierten Granulozyten (=PMN, Konzentration 10⁵/ml) und Thrombozyten (10⁶/ml) wird mit 1 µM FMLP und 1 U/ml Thrombin versetzt. Durch Zentrifugieren (10 min bei 1000 g) wird der Überstand gewonnen und in das obere Flüssigkeitskompartiment der Versuchsapparatur eingebracht. Dadurch erhöht sich - als Ausdruck der Kontraktion der venoiären Endothelzellen - die hydraulische Konduktivität gegenüber dem Ausgangswert (Nr. 1 in Tabelle 4) um ca. 1600 % (Nr. 10 in Tabelle 4). Bei Zusatz von 50 µM Apigenin (Nr. 11 in Tabelle 4) wird unter sonst gleichen Bedingungen die hydraulische Konduktivität nahezu auf den Ausgangswert zurückgeführt, d. h. die Kontraktion der venolären Endothelzellen wird fast vollständig gehemmt.

Isolierung von Endothelzellen venolären Ursprungs aus dem Koronarsystem von Meerschweinchenherzen

Zur Dissoziation des Myokardgewebes werden zunächst mit Krebs-Henseleitlösung (KHL) nach Langendorff bei 120 mmHg 10 min perfundierte Meerschweinchenherzen 35 min lang mit einem in KHL angesetzten Proteasegemisch perfundiert (42 mg Kollagenase D + 60 mg Dispase II + 5 mg Trypsin + 58 mg Serumalbumin aus Rinderblut, gelöst in 42 ml Ca²⁺-freier KHL; 1 ml/min; Kollagenase und Dispase: Boehringer, Mannheim, Trypsin: 1:250, Serva, Heidelberg). Nach Dissektion des Ventrikelgewebes und dessen mechanischer Zerkleinerung in ca. 2*2*2 mm Stücke wird eine vollständige Dissoziation des Myokards in Muskelzellen und Gefäße unter leichtem Schütteln über 60 min bei 37°C in 19 ml einer analog frisch angesetzten Proteaselösung erreicht. Die erhaltene Zell- bzw. Gefäßsuspension wird 3 mal vorsichtig durch eine 20 G Kanüle gesaugt, dann über ein Nylonnetz der Maschenweite 200 µm filtriert, und mit 11 ml einer isotonischen Percollösung ("Percoll" von Pharmacia, Upsala, Schweden) versetzt. Eine anschließende 10 minütige Zentrifugation bei 325 g führt zur weitgehend selektiven Sedimentation der vollständig dissoziierten venolären Endothelzellen. Das erhaltene Sediment wird noch zweimal mit frischer Percollösung derselben Dichte gewaschen.

Eine weitere Anreicherung der venolären Endothelzellen erfolgt durch Zentrifugation über einen linearen Percolldichtegradienten (1.04 - 1.06 g/cm³) im 80 ml-Separationsgefäß der Zentrifuge Z 323 (Hermle, Wehingen).

Die mikroskopisch homogen erscheinende Fraktion der Endothelzellen (Dichtebereich um 1.055 g/cm³) wird dreimal mit Kulturmedium gewaschen (Dulbecco Minimal Essential Medium unter Zusatz von 10% FCS, 1mM Glutamin, Penicillin 200 IE/ml und 20 µg/ml Streptomycin) und schließlich im gleichen Medium ausgesät. Nach Formierung geschlossener, durch ihre typische Architektur klar abgrenzbarer Endothelkolonien (nach ca. 3-wöchiger Kultur unter Standardbedingungen) werden diese unter mikroskopischer Kontrolle mit Hilfe steriler Glasspatel nochmals nachgereinigt.

### Etablierung konfluenter Endothelschichten auf Polycarbonatfiltern

### Einspannen der Polycarbonatfolien

Nach dem Zuschneiden der Polycarbonatfolien (Porendurchmeser 0,4 µm, Fa. Nucleopore, Pleasanton, USA) werden diese mit 100 % Ethanol getränkt, auf das Formteil der Filterspannvorrichtung aufgelegt, mit dem Stahlring umrandet und durch Aufdrücken des Halteringes im Verein mit dem Stahlring trommelfellartig ausgespannt (vgl. Abb. 1). Nach oben herausragende, überschüssige Polycarbonatfolie wird mit einem Skalpell entfernt.

### Vorbereitung der Filter

Die fertig montierten Filtersysteme werden in 0,5%iger Essigsäure 30 min bei 50 °C gereinigt, anschließend 5 mal mit bidestilliertem Wasser gespült und dann im Autoklaven in einem mit bidestilliertem Wasser gefüllten Becherglas sterilisiert. Anschließend erfolgt die Inkubation mit einer 0,4%igen Gelatinelösung für 60 min bei 100°C, danach weitere 60 min in 2,5% wässriger Glutaraldehydlösung bei Zimmertemperatur. Danach wird 3 mal mit bidestilliertem Wasser gewaschen und weitere 60 min in der auf 25 °C abgekühlten 0,4%igen Gelatinelösung inkubiert. Nach einer erneuten 60 minütigen Fixation in der 2,5%igen Glutaraldehydlösung werden die Filter 5 mal mit PBS gewaschen und schließlich in eine 20 mM L-Glutaminlösung zur Enttoxifikation überführt (48 h bei 4°C). Anschließend werden die Filter 5 mal mit PBS gewaschen, in sterile 35 mm Kulturschalen mit PBS überführt und 3 h mit UV-Licht nachsterilisiert. Danach werden die Filterringe für 24 h in steriles Kulturmedium eingelegt. Alle weiteren Handhabungen der Filtersysteme werden unter strikt sterilen Bedingungen vorgenommen.

### Zellkultur

Nach dem Absaugen des Mediums werden die Filtersysteme 3 h mit Fibronektinlösung (25µg/ml in PBS) inkubiert. Anschließend werden sie in neue Kulturschalen mit Kulturmedium überführt. Nach dem Absaugen des Mediums bis zum Niveau der Polycarbonatmembran wird das vorgesehene, in 800 µl Kulturmedium suspendierte Zellinokulum gleichmäßig auf der Membran verteilt; dabei werden jeweils 2,5*10⁵ Endothelzellen eingesetzt. Nach 3h wird jede Filtereinheit wieder in eine frische Kulturschale mit Medium überführt, um diejenigen Zellen, die sich nach 3 h noch nicht angeheftet haben, zu entfemen. Die sich anschließende Etablierung konfluenter Zellagen erfolgt über 6 Tage im Gewebekulturschrank, wobei alle 48 h die Hälfte des Kulturmediums erneuert wird.

### Plasmasalzlösung:

142 mM NaCl; 5,4 mM KCl; 1 mM NaH₂PO₄; 0,8 mM MgSO₄; 5,5 mM Glucose; Lösungsmittel: bidest. H₂O; Albuminzusatz: 0,1% (w/v); der pH-Wert der endgültigen Lösung wird mit NaOH auf 7,4 eingestellt.

### Isolierung hochgereinigter PMN:

Anwendung von "Polymorphoprep" nach den Vorschriften der Herstellerfirma Nycomed Pharma (Oslo, Norwegen); Nachreinigung der bereits stark angereicherten PMN-Bande durch dreimaliges Waschen mit 60%iger, isotonischer Percollverdünnung ("Percoll" von Pharmacia, Uppsala, Schweden).

### Verzeichnis der zitierten Literatur:

1 Reimer KA, Murry CE, Richard VJ (1989). J. Mol. Cell Cardiol. 21: 1225-1239
2 Reithart B, Jamieson SW (1990). In: Heart and heart-lung transplantation - orthotopic and heterotopic techniques. Verlag R. S. Schulz, Percha, pp. 43-60
3 Oldfield GS, Commerford PJ, Opie LH (1986). J. Thorac. Cardiovasc. Surg. 91(6): 874-8
4 Buckberg GD (1990). Ann. Thorac. Surg. 50(2): 175-7
5 Chiba Y, Muraoka R, Ihaya A, Morioka K, Sasaki M, Uesaka T (1993). Cardiovasc. Surg. 1(4): 530-6
6
   a) Buckberg GD (1990). J. Thorac. Cardiovasc. Surg. 100: 461-462
   b) Buckberg GD (1991). J. Thorac. Cardiovasc. Surg. 102: 895-903
7
   a) H. Lahaun, H. Perucker, in: Methoden der Organischen Chemie (Ed. E. Müller), p. 962. Thieme, Stuttgart, 1975
   b) Hertog MGL et al. (1993). Lancet 342:1007-1011
   c) Harsteen B (1983). Biochem. Pharmacol. 32:1141-1148
8 Neumann HAM, Carlsson K, Brom GHM (1992). Eur. J. Clin. Pharmacol. 43:423-426
9 Frostad AB (1977). Clin. Allergy 7: 347

## Patentansprüche

1. Wirkstoffgemisch als Pharmazeutikum, insbesondere zur Prophylaxe und Therapie von ischämischen Organschäden und Reperfusionssyndromen, umfassend mindestens einen Hemmstoff der Kontraktilität von venolären Endothelzellen und mindestens einen Hemmstoff der Cyclooxygenase 1, wobei der Hemmstoff der Kontraktilität venolärer Endothelzellen eine Benzopyron-Verbindung, ausgenommen eine blutgerinnungshemmende Benzopyron-Verbindung, ist.

2. Wirkstoffgemisch nach Anspruch 1, **dadurch gekennzeichnet, daß** der Hemmstoff der Cyclooxygenase 1 mindestens ein nichtsteroidales Antiphlogistikum ist.

3. Wirkstoffgemisch nach einem der Ansprüche 1 oder 2, umfassend zusätzlich mindestens eine pharmakologisch verträgliche, leicht oxidierbare Verbindung.

4. Wirkstoffgemisch nach Anspruch 3, **dadurch gekennzeichnet, daß** die leicht oxidierbare Verbindung Ascorbinsäure ist.

5. Wirkstoffgemisch nach einem der Ansprüche 1 bis 4, umfassend zusätzlich Harnsäure.

6. Wirkstoffgemisch nach einem der Ansprüche 1 bis 5, umfassend zusätzlich Inosin.

7. Wirkstoffgemisch nach einem der Ansprüche 1 bis 6, umfassend zusätzlich Asparaginsäure und Glutaminsäure.

8. Wirkstoffgemisch nach einem der Ansprüche 1 bis 7, umfassend zusätzlich Arginin.

9. Wirkstoffgemisch nach einem der Ansprüche 1 bis 8, umfassend zusätzlich eine pharmakologisch verträgliche Puffersubstanz zur Einstellung auf einen pH-Wert von etwa 7,0 bis 7,5.

10. Wirkstoffgemisch nach einem der Ansprüche 1 bis 9, umfassend zusätzlich eine normokaliämische Histidin-gepufferte physiologische Kochsalzlösung.

11. Wirkstoffgemisch nach einem der Ansprüche 1 bis 10, umfassend zusätzlich eine hyperkaliämische wässerige Lösung.

12. Wirkstoffgemisch nach Anspruch 1, umfassend als Benzopyron-Verbindung eine Verbindung ausgewählt aus der Gruppe bestehend aus einem Flavonol, Isoflavonol, Flavon, Flavonon, Isoflavonon, Isoflavon, Benzo-γ-pyron, Cumarin, Authocyanidin. Chalchon und Catechin und deren physiologisch verträglichen Derivaten.

13. Wirkstoffgemisch nach Anspruch 12, **dadurch gekennzeichnet, daß** die Benzopyron-Verbindung Trihydroxyethylrutosid ist.

14. Wirkstoffgemisch nach Anspruch 2, umfassend als nichtsteroidales Antiphlogistikum eine Verbindung ausgewählt aus der Gruppe bestehend aus einem Salicylat, einem Propionsäure-Derivat, einem Essigsäure-Derivat, einem Fenaminsäure-Derivat, einem Diphenylcarbonsäure-Derivat und einem Oxicam.

15. Wirkstoffgemisch nach Anspruch 14, **dadurch gekennzeichnet, daß** das nichtsteroidale Antiphlogistikum eine Verbindung ausgewählt aus der Gruppe bestehend aus Acetylsalicylsäure, Ibuprofen, Indometacin und Diclophenac ist.

16. Wirkstoffgemisch nach Anspruch 13 und 15, umfassend Trihydroxyethylrutosid und Acetylsalicylsäure.

17. Verwendung eines Wirkstoffgemisches nach einem der Ansprüche 1 bis 16 zur Herstellung einer Pharmazeutikum Zusammensetzung zur Prophylaxe oder Therapie von ischämischen Organschäden und Reperfusionssyndromen.

## Claims

1. Mixture of active substances as a pharmaceutical, in particular for the prophylaxis and therapy of ischemic organ damages and reperfusion syndromes, comprising at least one inhibitor of the contractility of venular endothelial cells and at least one inhibitor of cyclooxygenase 1, wherein the inhibitor of the contractility of the venular endothelial cells is a benzopyrone compound, excluding a blood coagulation inhibiting benzopyrone compound.

2. Mixture of active substances according to claim 1, **characterized in that** the inhibitor of the cyclooxygenase 1 is at least one non-steroidal antiphlogistic.

3. Mixture of active substances according to claim 1 or 2, further comprising at least one pharmacologically acceptable compound that is easily oxidised.

4. Mixture of active substances according to claim 3, **characterized in that** the compound that is easily oxidised is ascorbic acid.

5. Mixture of active substances according to any one of claims 1 to 4, further comprising uric acid.

6. Mixture of active substances according to any one of claims 1 to 5, further comprising inosine.

7. Mixture of active substances according to any one of claims 1 to 6, further comprising aspartic acid and glutamic acid.

8. Mixture of active substances according to any one of claims 1 to 7, further comprising arginine.

9. Mixture of active substances according to any one of claims 1 to 8, further comprising a pharmacologically acceptable buffer substance for adjusting to a pH value of about 7.0 to 7.5.

10. Mixture of active substances according to any one of claims 1 to 9, further comprising a normokalemic histidine buffered physiological saline solution.

11. Mixture of active substances according to any one of claims 1 to 10, further comprising a hyperkalemic aqueous solution.

12. Mixture of active substances according to claim 1, comprising as the benzopyrone compound a compound selected from the group consisting of a flavonol, isoflavonol, flavone, flavorione, isoflavonone, isoflavone, benzo-γ-pyrone, coumarin, anthocyanidin, chalchone, D-catechol and the physiologically acceptable derivatives thereof.

13. Mixture of active substances according to claim 12, **characterized in that** the benzopyrone compound is trihydroxyethylrutin.

14. Mixture of active substances according to claim 2, comprising a compound selected form the group consisting of a salicylate, a propionic acid derivative, an acetic acid derivative, a phenamin acid derivative, a diphenylcarboxylic acid derivative and an oxicam as non-steroidal antiphlogistic.

15. Mixture of active substances according to claim 14, **characterized in that** the non-steroidal antiphlogistic is a compound selected from the group consisting of acetylsalicylic acid, ibuprofen, indometacin and diclophenac.

16. Mixture of active substances according to claim 13 and 15, comprising trihydroxyethylrutin and acetylsalicylic acid.

17. Use of a mixture of active substances according to any one of claims 1 to 16 for the manufacture of a pharmaceutical composition for the prophylaxis or therapy of ischemic organ damages and reperfusion syndromes.

## Revendications

1. Mélange de substances actives à usage de produit pharmaceutique, en particulier pour la prophylaxie et la thérapie de lésions organiques ischémiques et de syndromes de reperfusion, comprenant au moins un inhibiteur de la contractilité des cellules endothéliales des veinules et au moins un inhibiteur de cyclooxygénase 1, tandis que l'inhibiteur de la contractilité des cellules endothéliales des veinules est un composé de benzopyrone, à l'exception d'un composé de benzopyrone ralentissant la circulation sanguine.

2. Mélange de substances actives selon la revendication 1, **caractérisé en ce que** l'inhibiteur de cyclooxygénase 1 est au moins un anti-inflammatoire non-stéroïdien.

3. Mélange de substances actives selon l'une des revendications 1 ou 2, comprenant en outre au moins un composé facilement oxydable, pharmaceutiquement acceptable.

4. Mélange de substances actives selon la revendication 3, **caractérisé en ce que** le composé facilement oxydable est l'acide ascorbique.

5. Mélange de substances actives selon l'une des revendications 1 à 4, comportant en outre de l'acide urique.

6. Mélange de substances actives selon l'une des revendications 1 à 5, comportant en outre de l'inosine.

7. Mélange de substances actives selon l'une des revendications 1 à 6, comportant en outre de l'acide asparagique et de l'acide glutamique.

8. Mélange de substances actives selon l'une des revendications 1 à 7, comportant en outre de l'arginine.

9. Mélange de substances actives selon l'une des revendications 1 à 8, comportant en outre une substance tampon pharmaceutiquement acceptable, pour l'ajustement à une valeur de pH d'environ 7,0 à 7,5.

10. Mélange de substances actives selon l'une des revendications 1 à 9, comportant en outre une solution physiologique de chlorure de sodium tamponnée à l'histidine normokaliémique.

11. Mélange de substances actives selon l'une des revendications 1 à 10, comportant en outre une solution aqueuse hyperkaliémique.

12. Mélange de substances actives selon la revendication 1, comportant comme composé de benzopyrone un composé choisi dans le groupe consistant en flavonol, isoflavonol, flavone, flavonone, isoflavonone, isoflavone, benzo-γ-pyrone, coumarine, anthocyanidine, chalcone et catéchine et leurs dérivés physiologiquement acceptables.

13. Mélange de substances actives selon la revendication 12, **caractérisé en ce que** le composé de benzopyrone est le trihydroxyéthylrutoside.

14. Mélange de substances actives selon la revendication 2, comportant comme anti-inflammatoire non-stéroïdien un composé choisi dans le groupe consistant en un salicylate, un dérivé d'acide propionique, un dérivé d'acide acétique, un dérivé d'acide fénamique, un dérivé d'acide diphénylcarboxylique et un oxicam.

15. Mélange de substances actives selon la revendication 14, **caractérisé en ce que** l'anti-inflammatoire non-stéroïdien est un composé choisi dans le groupe consistant en l'acide acétylsalicylique, l'ibuprofène, l'indométacine et le diclophénac.

16. Mélange de substances actives selon la revendication 13 et 15, comportant du trihydroxyéthylrutoside et de l'acide acétylsalicylique.

17. Utilisation d'un mélange de substances actives selon l'une des revendications 1 à 16 pour la préparation d'une composition pharmaceutique pour la prophylaxie ou la thérapie de lésions organiques ischémiques et de syndromes de reperfusion.
